# EUROPÄISCHE PATENTSCHRIFT

(11) **EP 2 217 183 B1**
(45) Veröffentlichungstag und Bekanntmachung des Hinweises auf die Patenterteilung: **19.02.2014**
(21) Anmeldenummer: 08851318.9
(22) Anmeldetag: 13.11.2008
(51) Int. Cl.: A61F 5/11

(54) **VORRICHTUNG ZUR VORNAHME VON NAGELKORREKTUREN**
DEVICE FOR CARRYING OUT NAIL CORRECTIONS
DISPOSITIF POUR EXÉCUTER DES CORRECTIONS SUR DES ONGLES

(30) Priorität: 23.11.2007 DE 102007056614
(43) Veröffentlichungstag der Anmeldung: 18.08.2010
(73) Patentinhaber: Stolz, Bernd, 92224 Amberg (DE)
(72) Erfinder: Stolz, Bernd, 92224 Amberg (DE)
(74) Vertreter: Bauerschmidt, Peter
(86) Internationale Anmeldenummer: PCT/EP2008/009683
(87) Internationale Veröffentlichungsnummer: WO 2009/065533

(56) Entgegenhaltungen:
- BE-A- 457 090
- CH-A- 268 681
- US-A- 3 032 032

## Beschreibung

Die Erfindung richtet sich auf eine Vorrichtung zur Vornahme von Nagelkorrekturen, insbesondere bei einem eingewachsenen, zu stark gekrümmten Nagel, umfassend wenigstens einen sich in einer Längsrichtung erstreckenden Kunststoffstreifen.

Eine derartige Vorrichtung ist bekannt aus EP 0 282 645 A1. Dieser vorbekannte Korrekturstreifen wird vollflächig mit der Oberseite des zu korrigierenden Nagels verklebt, wozu er der Außenkontur des Nagels folgend gebogen werden muss. Durch diese Auslenkung entsteht eine elastische Rückstellkraft, die insbesondere auf die Zehennagelränder wirkt und diese hochhebt. Eine ähnliche Vorrichtung wird in der DE 197 11 923 A1 beschrieben, wobei die dort offenbarten Korrekturstreifen nicht aus Kunststoff, sondern aus einem superelastischen Material, wie z. B. einer Nickel-Titan-Legierung, bestehen.

Nachteilig an diesen erprobten und an sich sehr wirksamen Vorrichtungen ist, dass die Handhabung von Schnellklebstoffen ein hohes Maß an Aufmerksamkeit erfordert, da aufgrund von deren hoher Klebkraft die Gefahr besteht, dass die Finger der handhabenden Person verkleben oder der Klebstoff anderweitig Schäden anrichtet.

Beispielsweise aus der DE 10 2006 018 987 A1 sind auch schon Vorrichtungen aus Metall bekannt, welche seitliche Haken aufweisen, die jeweils einen Seitenrand des zu korrigierenden Nagels um- und untergreifen, wobei dann mittels einer Art Spannschraube eine einstellbare Spannkraft aufgebracht wird. Der Nachteil dieser Vorrichtungen besteht darin, dass sie, wenn sie in Schuhen getragen werden, als unangenehm empfunden werden und auch beim An- und Ausziehen von Strümpfen sehr störend wirken. Deshalb werden solche Vorrichtungen insbesondere wegen der vergleichsweise voluminösen Spannteileinheit oft mit einem Verband oder einem Pflaster abgedeckt, wodurch der Tragekomfort aber weiter beeinträchtigt wird. Darüber hinaus ist der Aufbau relativ kompliziert, was diese bekannten Vorrichtungen auch kostspielig macht.

In der BE 457 090 A wird eine Nagelkorrekturvorrichtung gemäß dem Oberbegriff von Anspruch 1 beschrieben, die aus einem elastischen Kautschuk-Band besteht, das an seinen Längsenden mit Haken versehen ist.

Hiervon ausgehend liegt der Erfindung die Aufgabe zugrunde, eine gattungsgemäße Vorrichtung zu schaffen, welche einfach sowohl in der Herstellung als auch in der Handhabung ist, das Hantieren mit Klebstoff vermeidet und gleichwohl eine Wirksamkeit aufweist, die derjenigen der bekannten Kunststoffstreifen entspricht bzw. diesen noch überlegen ist.

Diese Aufgabe wird erfindungsgemäß dadurch gelöst, dass der Kunststoffstreifen gummi- und längselastisch ist und an seinen beiden in Längsrichtung gesehenen Längsenden jeweils einen Haken zum Um- und Untergreifen eines Nagelrandes des zu korrigierenden Nagels aufweist, wobei der Kunststoffstreifen dehnbar und bei eingehakten Haken in seiner Länge an eine Nagelbreite des zu korrigierenden Nagels anpassbar ist, so dass eine elastizitätsbedingte Rückstellkraft eine automatisch an einen Krümmungsgrad des zu korrigierenden Nagels angepasste Aufrichtkraft bewirkt. Der Kunststoff der Haken ist härter und formbeständiger als der des Kunststoffstreifens.

Zum Applizieren einer erfindungsgemäßen Vorrichtung wird zunächst einer der beiden endseitigen Haken unter den seitlichen Außenrand des zu korrigierenden Zehennagels eingehängt. Dann wird der Kunststoffstreifen gespannt und der Haken am anderen Ende wird unter den gegenüberliegenden Zehennagelrand eingehängt. Dabei wird der Kunststoffstreifen gedehnt und an die Nagelbreite angepasst. Richtet sich der Nagel aufgrund der Korrekturvorrichtung im Laufe der Behandlungszeit wieder etwas auf, reduziert sich die Dehnung des elastischen Kunststoffstreifens, wodurch auch die Rückstellkraft und damit die Aufrichtkraft etwas geringer werden. Die erfindungsgemäße Vorrichtung passt sich also automatisch an die geänderten Gegebenheiten während der Behandlung an. Ein manuelles Nachspannen oder Lockern oder sogar ein Austausch der Korrekturvorrichtung gegen eine andere ist nicht erforderlich.

Aus dem Vorstehenden wird deutlich, dass der Kunststoffstreifen insbesondere ein Untermaß gegenüber der Breite des Zehennagels aufweist, damit durch die elastische Dehnung eine Spannkraft und damit eine Rückstellwirkung auf die Ränder des Zehennagels geschaffen wird.

Um diese Rückstellkraft zu optimieren oder einstellbar zu machen, kann der Kunststoffstreifen unterschiedliche Längen aufweisen und/oder unterschiedliche Breiten. Es können auch mehrere derartige Kunststoffstreifen parallel zueinander angelegt werden.

Es ist vorgesehen, dass Kunststoffstreifen und Haken gemeinsam aus Kunststoff gespritzt sind. So sind die Haken und der Kunststoffstreifen als ein gemeinsames Kunststoff-Spritzgussteil ausgeführt, das mittels eines Zweikomponenten-Spritzgussverfahrens (= 2K-Verfahren) hergestellt ist. Dabei wird für den gummielastischen Kunststoffstreifen ein erster Kunststoff und für die Haken ein zweiter Kunststoff im Rahmen eines gemeinsamen Herstellungsprozesses in eine einzige Spritzgussform eingebracht. Dies erfolgt gleichzeitig oder sukzessive, jedenfalls aber noch ehe der zuerst eingebrachte Kunststoff vollständig ausgehärtet ist. Infolge dieses günstigen Herstellungsprozesses resultiert eine sehr feste Verbindung zwischen dem Kunststoffstreifen und den Haken. Eine gesonderte Verklebung ist dann nicht erforderlich.

In jedem Fall ist eine einfache Applikation gewährleistet und der Gebrauch von Klebstoff wird vermieden.

Vorzugsweise besteht der Kunststoffstreifen aus einem Elastomer.

Außerdem ist es so insbesondere möglich, die Haken aus einem relativ harten, formbeständigen Kunststoff herzustellen, der einen festen, dauerhaften Halt am Rand des Zehennagels gewährleistet.

Um insbesondere bei einer einstückigen Ausführungsform einerseits eine hinreichende Haltekraft der Haken und andererseits eine ausreichende Elastizität des Kunststoffstreifens zu erreichen, kann der Kunststoffstreifen mit Materialschwächungen im Kunststoffstreifen, wie zum Beispiel Einbuchtungen, versehen sein. Der Kunststoffstreifen kann derartige Materialschwächungen aber auch unabhängig von dem verwendeten Herstellungsverfahren und unabhängig von der Verbindung mit den Haken aufweisen, beispielsweise zur Einstellung einer gewünschten Längselastizität. Die Längselastizität lässt sich so erhöhen.

Gemäß einer weiteren bevorzugten Ausgestaltung sind zwischen den beiden endseitigen Haken mehrere senkrecht zur Längsrichtung nebeneinander, also insbesondere parallel zueinander, angeordnete jeweils gummiund längselastische Kunststoffstreifen vorgesehen. Auch dadurch lässt sich die Längselastizität einstellen. Insbesondere kann die Aufrichtkraft gesteigert werden, indem die Anzahl der parallel nebeneinander angeordneten Kunststoffstreifen erhöht wird.

Gemäß einer weiteren bevorzugten Ausgestaltung ist die Vorrichtung mehrfach verwendbar und/oder desinfizierbar ausgeführt. Dies schont Ressourcen. Außerdem erhöht dies den Wert der Vorrichtung für den Anwender.

Gemäß einer weiteren bevorzugten Ausgestaltung können die Haken aus einem nachgiebigen Material bestehen. Dadurch ist die Vorrichtung flexibler in der Anwendung. Außerdem erreicht man dadurch eine geringere Beeinträchtigung des Anwenders. Etwas nachgiebigere Haken verursachen weniger Schmerzen am Nagelbett als sehr harte Haken.

Gemäß weiteren bevorzugten Ausgestaltungen haben die Haken jeweils einen U-förmigen Längsquerschnitt mit eckigem oder abgerundetem Übergang zwischen den U-Schenkeln und dem U-Boden oder einen Längsquerschnitt in Form eines Kreisringsektors, der sich insbesondere über einen Umfangswinkel von mehr als 180° erstreckt. Die zuerst genannte Ausgestaltung vermittelt einen sehr guten an die jeweiligen Gegebenheiten angepassten Halt, während die zweite Ausgestaltung eine flexiblere Anwendung ermöglicht, da ein und dieselbe Vorrichtung für unterschiedlich dicke Nägel verwendet werden kann. Durch ein Aufspreizen des Kreisringsektors erreicht man eine Anpassung an unterschiedliche Nagelstärken. Dadurch sinkt die vorzuhaltende Vielzahl an unterschiedlichen Korrekturvorrichtungen.

Weitere Merkmale, Vorteile und Einzelheiten der Erfindung ergeben sich aus der nachfolgenden Beschreibung von Ausführungsbeispielen anhand der Zeichnung. Es zeigt:
- Fig. 1: ein erstes Ausführungsbeispiel einer Vorrichtung zur Nagelkorrektur mit einem längselastischen Streifen und mit an dessen Längsenden angebrachten U-förmigen Haken in einer schematischen Seitenansicht,
- Fig. 2: ein zweites Ausführungsbeispiel einer Vorrichtung zur Nagelkorrektur mit mehreren nebeneinander liegenden längselastischen Streifen in einer schematischen Draufsicht,
- Fig. 3: ein drittes Ausführungsbeispiel einer Vorrichtung zur Nagelkorrektur mit einem mit Materialschwächungen versehenen längselastischen Streifen in einer schematischen Draufsicht, und
- Fig. 4: ein viertes Ausführungsbeispiel einer Vorrichtung zur Nagelkorrektur mit einem kreisringsektorförmigen Haken in einer ausschnittsweisen schematischen Seitenansicht und im applizierten Zustand.

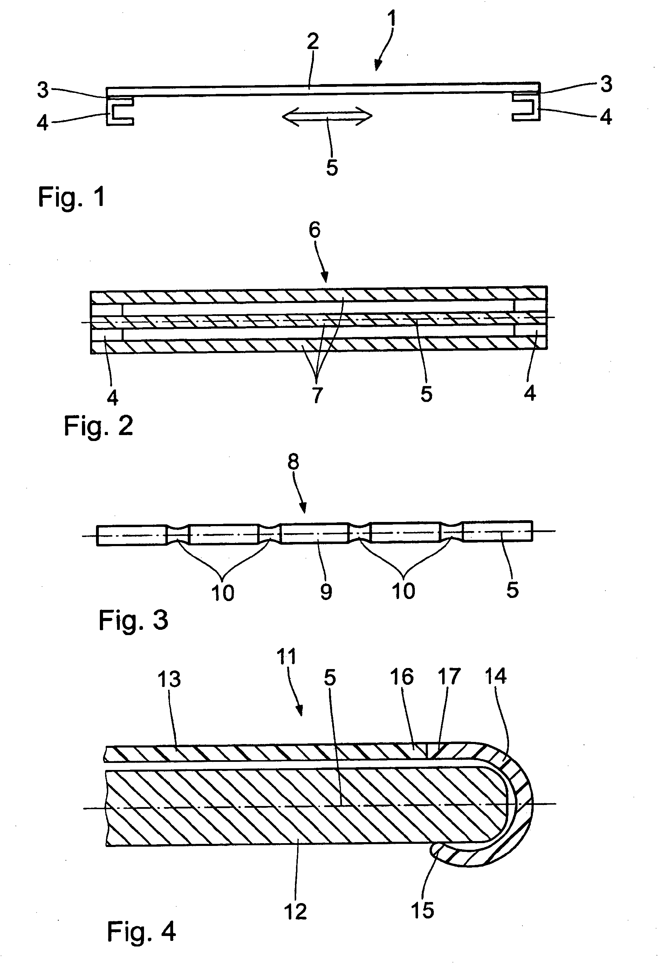

Einander entsprechende Teile sind in den Fig. 1 bis 4 mit denselben Bezugszeichen versehen.

Eine in Fig. 1 dargestellte erfindungsgemäße Nagelkorrekturvorrichtung 1 umfasst einen zentralen länglichen Streifen 2 aus einem gummielastischen elastomeren Kunststoff, an dessen beiden Längsenden jeweils über eine Klebstoffschicht 3 ein U-förmiger, nach innen offener Haken 4 aus einem härteren Kunststoff angebracht ist. Der längselastische Streifen 2 erstreckt sich in einer Längsrichtung 5. Der Streifen 2 kann in dieser Längsrichtung 5 gedehnt werden, so dass er an eine Breite des zu korrigierenden Nagels angepasst ist. Aufgrund der Dehnung ergibt sich dann eine elastizitätsbedingte Rückstellkraft, die eine Aufrichtkraft auf den zu korrigierenden gekrümmten Nagel bewirkt. Wegen der Gummi- und Längselastizität des Streifens 2 passt sich diese auf den Nagel ausgeübte Aufrichtkraft während der Behandlungsdauer an den sich ggf. verändernden Krümmungsgrad des Nagels insbesondere automatisch an.

Es kann ein Satz an Nagelkorrekturvorrichtungen 1 mit jeweils anderer Länge vorgesehen sein. Die in diesem Satz vorrätig gehaltenen Streifenlängen können z. B. 12 mm, 14 mm, 16 mm, 18 mm, 20 mm und 22 mm betragen. Dann kann die für den jeweiligen Anwendungsfall am besten geeignete Nagelkorrekturvorrichtung 1 ausgewählt werden. Wenn der zu korrigierende Nagel z. B. eine Nagelbreite von 18 mm hat, wird die Nagelkorrekturvorrichtung 1 mit einer demgegenüber kleineren Streifenlänge insbesondere von 14 mm oder 16 mm appliziert. Die resultierende Dehnung um 4 mm bzw. 2 mm bewirkt dann die gewünschte Aufrichtkraft. Vorzugsweise ist der Streifen 2 im applizierten Zustand um bis zu etwa 5 mm oder um bis zu etwa 30 %, insbesondere um bis zu etwa 15 % seiner im nicht applizierten Zustand gegebenen Ausgangslänge gedehnt.

In Fig. 2 ist ein anderes Ausführungsbeispiel einer Nagelkorrekturvorrichtung 6 gezeigt. Sie umfasst im Unterschied zu der Nagelkorrekturvorrichtung 1 gemäß Fig. 1 insgesamt drei parallel nebeneinander liegende wiederum gummi- und längselastische und sich in Längsrichtung 5 erstreckende Streifen 7 aus einem Kunststoff. Die Anzahl von drei Streifen 7 ist beispielhaft zu verstehen. Grundsätzlich können auch mehr oder weniger Streifen 7 nebeneinander angeordnet sein. An jedem Längsende sind die drei Streifen 7 gemeinsam mit jeweils einem der Haken 4 verbunden. Aufgrund der Parallelanordnung mehrerer Streifen 7 lässt sich die Längselastizität und damit die Aufricht- oder Zugkraft auf den Nagel sehr genau und in gewissen Grenzen auch variabel einstellen. So kann nämlich bei einem eingetretenen Behandlungsteilfortschritt einer oder mehrere der Streifen 7 durchgeschnitten werden, um so eine für die Restbehandlung nur noch benötigte geringere Aufricht- oder Zugkraft einzustellen.

In Fig. 3 ist ein Ausführungsbeispiel einer Nagelkorrekturvorrichtung 8 gezeigt, bei der ein Kunststoffstreifen 9 mit Materialschwächungen 10 versehen ist. Aufgrund der so gebildeten Bereiche mit geringerer Querschnittsfläche senkrecht zur Längsrichtung 5 kann die Längselastizität des Kunststoffstreifens 9 erhöht werden. Dann ist es möglich, den Kunststoffstreifen 9 und die Haken 4 als einstückiges Kunststoff-Spritzgussteil, auszuführen.

Der Ausschnitt gemäß Fig. 4 zeigt ein weiteres Ausführungsbeispiel einer Nagelkorrekturvorrichtung 11 im applizierten, d. h. im an einem zu korrigierenden Nagel 12 angebrachten, Zustand. Auch die Nagelkorrekturvorrichtung 11 setzt sich aus einem zentralen längselastischen Streifen 13 und zwei an den Längsenden des Streifens 13 angeformten Haken 14 zusammen. Die Haken 14, von denen in dem Ausschnitt gemäß Fig. 4 nur einer gezeigt ist, bestehen aus Kunststoff, aber aus einem anderen als der Streifen 13. Verglichen mit dem gummielastischen Streifen 13 sind die Haken 14 härter, aber immer noch zu einem gewissen Grad elastisch ausgebildet. Die Haken 14 haben in der Seitenansicht gemäß Fig. 4 oder in einer Querschnittsebene, die die Längsrichtung 5 enthält, in etwa eine Kreisringsektorform bzw. in etwa die Form eines offenen O. Der von den annähernd kreisringsektorförmigen Haken 14 überstrichene Umfangswinkelbereich ist größer als 180°. Wegen der gegebenen gewissen Elastizität bzw. Nachgiebigkeit der Haken 14 kann ein freies Hakenende 15 aufgebogen und damit ein Aufnahmebereich des Hakens 14 an die Dicke des Nagels 12 angepasst werden.

Der Streifen 13 und die Haken 14 sind mittels eines 2K-Spritzgussverfahrens hergestellt. Sie bilden ein gemeinsames Kunststoff-Spritzgussteil. Dadurch ergibt sich eine feste, insbesondere klebstofffreie Verbindung zwischen einem Streifenlängsende 16 und einem Kopplungsende 17 des Hakens 14. Diese Verbindung ist bei dem gezeigten Ausführungsbeispiel dadurch gebildet, dass die Stirnseiten des Streifens 13 am Streifenende 17 und des Hakens 14 am Kopplungsende 17 stumpf aneinander stoßen. Aufgrund des verwendeten 2K-Herstellungsverfahrens stellt sich an dieser Kopplungsstelle ein inniger Materialverbund zwischen den beiden Kunststoffen des Streifens 13 und des Hakens 14 ein.

Die Nagelkorrekturvorrichtungen 1, 6, 8 und 11 lassen sich desinfizieren. Sie können also nach dem Gebrauch so gesäubert werden, dass sie sich wieder verwenden lassen.

## Patentansprüche

1. Vorrichtung zur Vornahme von Nagelkorrekturen, insbesondere bei einem eingewachsenen, zu stark gekrümmten Nagel (12), umfassend wenigstens einen sich in einer Längsrichtung (5) erstreckenden Kunststoffstreifen (2; 7; 9; 13), wobei der Kunststoffstreifen (2; 7; 9; 13) gummi- und längselastisch ist und an seinen beiden in Längsrichtung (5) gesehenen Längsenden (16) jeweils einen Haken (4; 14) zum Um- und Untergreifen eines Nagelrandes des zu korrigierenden Nagels (12) aufweist, wobei der Kunststoffstreifen (2; 7; 9; 13) dehnbar und bei eingehakten Haken (4; 14) in seiner Länge an eine Nagelbreite des zu korrigierenden Nagels (12) anpassbar ist, so dass eine elastizitätsbedingte Rückstellkraft eine automatisch an einen Krümmungsgrad des zu korrigierenden Nagels (12) angepasste Aufrichtkraft bewirkt, **dadurch gekennzeichnet, daß** die Haken (4; 14) aus einem zweiten Kunststoff bestehen, der härter und formbeständiger ist als ein erster Kunststoff des Kunststoffstreifens (2; 7; 9; 13), wobei die Haken (14) und der Kunststoffstreifen (9; 13) als ein gemeinsames Kunststoff-Spritzgussteil ausgeführt sind, welches mittels eines Zweikomponenten-Spritzgussverfahrens hergestellt ist, bei dem für den gummielastischen Kunststoffstreifen (2; 7; 9; 13) der erste Kunststoff und für die Haken (14) der zweite Kunststoff im Rahmen eines gemeinsamen Herstellungsprozesses in eine einzige Spritzgussform eingebracht werden, so dass eine Verbindung zwischen dem Kunststoffstreifen (2; 7; 9; 13) und den Haken (14) resultiert.

2. Vorrichtung nach Anspruch 1, **dadurch gekennzeichnet, dass** der Kunststoffstreifen (2; 7; 9; 13) aus einem Elastomer besteht.

3. Vorrichtung nach einem der vorhergehenden Ansprüche, **dadurch gekennzeichnet, dass** Materialschwächungen (10) im Kunststoffstreifen (8) vorgesehen sind.

4. Vorrichtung nach einem der vorhergehenden Ansprüche, **dadurch gekennzeichnet, dass** zwischen den beiden endseitigen Haken (4) mehrere senkrecht zur Längsrichtung (5) nebeneinander angeordnete jeweils gummi- und längselastische Kunststoffstreifen (7) vorgesehen sind.

5. Vorrichtung nach einem der vorhergehenden Ansprüche, **dadurch gekennzeichnet, dass** sie mehrfach verwendbar ausgeführt ist.

6. Vorrichtung nach einem der vorhergehenden Ansprüche, **dadurch gekennzeichnet, dass** sie desinfizierbar ausgerührt ist.

7. Vorrichtung nach einem der vorhergehenden Ansprüche, **dadurch gekennzeichnet, dass** die Haken (4; 14) aus einem nachgiebigen Material bestehen.

8. Vorrichtung nach einem der vorhergehenden Ansprüche, **dadurch gekennzeichnet, dass** die Haken (4) jeweils einen U-förmigen Längsquerschnitt mit eckigem oder abgerundetem Übergang zwischen den U-Schenkeln und dem U-Boden haben.

9. Vorrichtung nach einem der Ansprüche 1 bis 7, **dadurch gekennzeichnet, dass** die Haken (14) jeweils einen Längsquerschnitt in Form eines Kreisringsektors haben.

10. Vorrichtung nach Anspruch 9, **dadurch gekennzeichnet, dass** sich der Kreisringsektor des Hakens (14) über einen Umfangswinkel von mehr als 180° erstreckt.

## Claims

1. Device for carrying out nail corrections, in particular in an ingrown nail (12), which is too severely curved, comprising at least one plastics material strip (2; 7; 9; 13) extending in a longitudinal direction (5), wherein the plastics material strip (2; 7; 9; 13) is rubbery-elastic and longitudinally elastic and, at its two longitudinal ends (16), viewed in the longitudinal direction (5), has a respective hook (4; 14) to engage around and underneath a nail edge of the nail (12) to be corrected, the plastics material strip (2; 7; 9; 13) being expandable and, when the hook (4; 14) is hooked in, being adaptable with respect to its length to a nail width of the nail (12) to be corrected, so that a restoring force due to elasticity brings about an upward force automatically adapted to a degree of curvature of the nail (12) to be corrected, **characterised in that** the hooks (4; 14) consist of a plastics material, which is harder and more dimensionally stable than a first plastics material of the plastics material strip (2; 7; 9; 13), wherein the hooks (14) and the plastics material strip (9; 13) are configured as a joint plastics material injection-moulded part which is produced by means of a two-component injection moulding method in which the first plastics material is introduced for the rubbery-elastic plastics material strip (2; 7; 9; 13) and the second plastics material is introduced for the hooks (14) into a single injection mould in the course of a joint production process, with the result that a connection between the plastics material strip (2; 7; 9; 13) and the hook (14) is produced.

2. Device according to claim 1, **characterised in that** the plastics material strip (2; 7; 9; 13) consists of an elastomer.

3. Device according to any one of the preceding claims, **characterised in that** material weakenings (10) are provided in the plastics material strip (8).

4. Device according to any one of the preceding claims, **characterised in that** a plurality of rubbery-elastic and longitudinally elastic plastics material strips (7) in each case arranged next to one another perpendicular to the longitudinal direction (5) are provided between the two end hooks (4).

5. Device according to any one of the preceding claims, **characterised in that** it can be used several times.

6. Device according to any one of the preceding claims, **characterised in that** it can be disinfected.

7. Device according to any one of the preceding claims, **characterised in that** the hooks (4; 14) consist of a resilient material.

8. Device according to any one of the preceding claims, **characterised in that** the hooks (4) each have a U-shaped longitudinal cross section with an angular or rounded transition between the U-sides and the U-base.

9. Device according to any one of claims 1 to 7, **characterised in that** the hooks (14) in each case have a longitudinal cross section in the shape of a ring sector.

10. Device according to claim 9, **characterised in that** the ring sector of the hook (14) extends over a peripheral angle of more than 180°.

## Revendications

1. Dispositif pour exécuter des corrections sur des ongles, notamment des ongles (12) incarnés, trop fortement recourbés, comprenant au moins un ruban (2 ; 7 ; 9 ; 13) en matière synthétique s'étendant dans le sens de la longueur (5), le ruban (2 ; 7 ; 9 ; 13) en matière synthétique possédant une élasticité caoutchoutique et une élasticité longitudinale, et présentant à chacune de ses deux extrémités longitudinales (16), vu dans le sens de la longueur (5), un crochet (4 ; 14) destiné à entourer et à accrocher par le dessous un bord d'ongle de l'ongle (12) devant être corrigé, la bande (2 ; 7 ; 9 ; 13) en matière synthétique étant extensible et pouvant être adaptée à une largeur d'ongle de l'ongle (12) devant être corrigé sur son étendue lorsque le crochet (4 ; 14) est accroché, de sorte qu'une force de rappel élastique conduit automatiquement à une force de redressement adaptée à un degré de courbure de l'ongle (12) devant être corrigé, **caractérisé en ce que** les crochets (4 ; 14) sont constitués d'une seconde matière synthétique qui est plus dure et davantage indéformable que la première matière synthétique de la bande (2 ; 7 ; 9 ; 13) en matière synthétique, les crochets (14) et la bande (9 ; 13) en matière synthétique étant conçus sous la forme d'une pièce de moulage par injection de matière synthétique commune, qui est fabriquée au moyen d'un procédé de moulage par injection à deux composants, dans lequel la première matière synthétique pour la bande (2 ; 7 ; 9 ; 13), à élasticité caoutchoutique, et la seconde matière synthétique pour les crochets (14) sont alimentées dans un moule de moulage par injection conjoint dans le cadre d'un procédé de fabrication commun, de sorte qu'il en résulte une combinaison entre la bande (2 ; 7 ; 9 ; 13) en matière synthétique et les crochets (14).

2. Dispositif selon la revendication 1, **caractérisé en ce que** la bande (2 ; 7 ; 9 ; 13) est constituée d'un élastomère.

3. Dispositif selon l'une des revendications précédentes, **caractérisé en ce que** des affaiblissements de matière (10) sont prévus dans la bande (8) en matière synthétique.

4. Dispositif selon l'une des revendications précédentes, **caractérisé en ce que** plusieurs bandes (7) en matière synthétique, à élasticité caoutchoutique et longitudinale, disposées les unes à côté des autres dans le sens de la longueur (5), sont prévues entre les deux crochets (4) terminaux.

5. Dispositif selon l'une des revendications précédentes **caractérisé en ce qu'il** est conçu pour une utilisation multiple.

6. Dispositif selon l'une des revendications précédentes, **caractérisé en ce qu'il** est conçu pour être désinfecté.

7. Dispositif selon l'une des revendications précédentes, **caractérisé en ce que** les crochets (4 ; 14) sont constitués d'un matériau flexible.

8. Dispositif selon l'une des revendications précédentes, **caractérisé en ce que** les crochets (4) possèdent chacun une section longitudinale en forme de U possédant une zone de transition en angle ou arrondie entre les jambages du U et la base du U.

9. Dispositif selon l'une des revendications 1 à 7, **caractérisé en ce que** les crochets (14) possèdent chacun une section longitudinale sous forme d'un secteur de couronne circulaire.

10. Dispositif selon la revendication 9, **caractérisé en ce que** le secteur de couronne circulaire du crochet (14) s'étend sur un angle inscrit supérieur à 180°.
